# NEW EUROPEAN PATENT SPECIFICATION

(11) **EP 0 135 315 B2**
(45) Date of publication and mention of the opposition decision: **26.04.1995**
(45) Mention of the grant of the patent: 18.07.1990
(21) Application number: 84305123.6
(22) Date of filing: 27.07.1984
(51) Int. Cl.: A61K 7/32

(54) **Antiperspirant creams**
Antitranspirant-Cremes
Crèmes anti-perspirantes

(30) Priority: 29.07.1983 US 518384
(43) Date of publication of application: 27.03.1985
(73) Proprietor: THE MENNEN COMPANY, Morristown New Jersey 07960 (US)
(72) Inventor: Kasat, Radhakrishna B., Belle Mead New Jersey 08502 (US)
(74) Representative: Hale, Stephen Geoffrey

(56) References cited:
- EP-A- 0 028 853
- EP-A- 0 037 011
- EP-A- 0 116 406
- CA-A- 1 099 645
- CA-A- 1 115 213
- CA-A- 1 146 867
- FR-A- 2 339 392
- FR-A- 2 501 042
- US-A- 4 083 956
- US-A- 4 280 994
- J.Soc. Cosmet. Chem. vol. 30, May-June 1979, p. 137-156
- Chemical Abstracts, vol. 94, abstract no. 214400v
- Cosmetics Science and Technology, NY 1957, pages 998, 1005-1013

## Description

This invention is directed to a creamy, heterogeneous, anhydrous antiperspirant product and a method for making it.

Antiperspirant compositions in the form of creams have heretofore been primarily oil-in-water or water-in-oil emulsions. Though these provide convenient vehicles for carrying and delivering the astringent, emulsions generally tend to produce undesirable sensations on the skin, such as a sticky, wet feeling.

Anhydrous antiperspirant creams essentially comprising a liquid organic emollient material, an inorganic clay thickening/suspending agent, a gel-promoting agent and an astringent are also known, for example from US-A-4,083,956. These also tend to produce undesirable sensations on the skin, will tend to remain on the skin and will cause stains on fabrics which come into contact with the product.

Another stable, essentially anhydrous antiperspirant cream comprising an antiperspirant agent and an oil absorbent homogeneously dispersed in a vehicle containing a volatile silicone and a clay suspending thickening agent in the form of a gel is disclosed in FR-A-2,501,042.

Antiperspirant stick products are also well-known in the prior art. For example, US-A-4,126,679 describes antiperspirant stick products made by suspending an astringent in a solid solution of volatile silicones and long chain alcohols.

Prior art methods for preparing antiperspirant compositions tend to vary depending on whether the desired product is a stick or a cream. The above-mentioned US-A-4,083,956, for example, describes a method of preparing the thixotropic creams described there. In the described method of preparation, the emollient and optional ingredients soluble therein are mixed together. The clay suspending/thickening agent is added and mixed with an agitator to provide a uniform composition. Gel-promoting and anti-syneresis agents then are added until gellation occurs. At that point, antiperspirant active ingredients are blended into the mixture and uniformly dispersed. Apparently, no heating of the ingredients is necessary or desirable to prepare the products.

In FR-A-2,501,042 the antiperspirant cream is prepared by forming a mixture of the vehicle containing the volatile silicone and the clay suspending/thickening agent in gel form, heating that mixture to 60-105°C until a viscous solution is formed, sequentially adding the antiperspirant agent and the oil absorbent to form a stable viscous mixture which may be homogenised in a warm porous state, perfume being added (if desired) after cooling to 43-66°C.

US-A-4,126,679 and US-A-4,049,792 describe methods for making the antiperspirant sticks described there. Briefly, the ingredients are mixed together and melted, the astringent is added and intermixed and the resulting uniform product is poured into the stick form, at a temperature which is above the normal solidification point, and is allowed to cool to the normal solidification point of the product, and further to room temperature, without further agitation or mixing.

It has now been found, quite unexpectedly, that a particularly acceptable, efficacious, creamy antiperspirant product may be made without the need to include a clay suspending/thickening agent characteristic of prior art products such as those disclosed in US-A-4,083,956 and FR-A-2,501,042. The product of the invention provides a dry feeling, imparts no sticky feeling whatever and does not cool the skin, as the prior art products do, after being applied thereto. Furthermore, it appears that the method employed in making the antiperspirant creams of the present invention is unexpectedly suitable to make the creams described herein.

The present invention provides a creamy, clay-free, heterogeneous, anhydrous antiperspirant composition comprising the following essential ingredients: about 30 to about 70% (all percentages are by weight of total product) of a volatile silicone product carrier, about 7 to about 30% of a gelling agent and about 12 to about 30% of a physiologically acceptable antiperspirant agent, and characterised in that it is obtainable by the steps of mixing and heating the carrier, the gelling agent, the antiperspirant agent and any optional ingredients except the fragrance (if any) to a temperature above the melting point of the gelling agent and then cooling the mixture below the normal solidification point of the product while still mixing or agitating the product or otherwise subjecting it to shear. It also provides a method of making such a composition.

The carrier, for example, can be any volatile, low viscosity silicone product, either cyclic or open-chain, or combinations thereof.

The gelling agent can be any of a number of compositions, including, for example, hydrogenated vegetable oil, hydrogenated castor oil, fatty acids, beeswax, paraffin wax, fatty alcohols, polyethylene or combinations thereof.

The antiperspirant agent can be any of the usual types of astringents, such as aluminum chlorohydrate or aluminum zirconium chlorohyhdrate.

Apart from the above-identified ingredients, i.e. the carrier, the gelling agent and the antiperspirant agent, it is desirable to include one or more optional ingredients in the creamy antiperspirant product of the invention. These optional ingredients together can comprise up to about 25% of the total product.

Useful optional ingredients include surfactants, fillers, emollients, fragrances and coloring agents, for example.

Surfactants can comprise up to about 5% of the total product and aid in preventing stains on clothing and in washing out any stains which do form.

Fillers can comprise up to about 20% of the total product and are normally less costly than the essential components of the product, thus reducing the overall cost.

Emollients can comprise up to about 15% of the total product, and, though not essential ingredients, are useful particularly when hydrogenated castor oil (also known as castor wax) is used as the gelling agent.

Fragrances can comprise up to about 1% of the total product.

Coloring agents can be added as desired.

The method of the invention comprises, very briefly, heating and thoroughly mixing all ingredients, then cooling the resultant mixture below the normal solidification point thereof, while subjecting the product to continuous shearing, agitation or mixing action. This process produces the creamy heterogeneous, anhydrous antiperspirant product of the invention. In practice, it has been found that a commercially available device called a Votator Scraped Surface Heat Exchanger, available from several sources, for example Chemetron Corporation of Louisville, Kentucky, is useful, but not necessary, in practicing the method of the invention.

As stated above, one essential ingredient of the invention is a carrier, which comprises a volatile silicone product. The preferred volatile silicone products according to the invention are in general polydimethylcyclosiloxanes having 3 to 6 silicon atoms and linear polydimethyl siloxanes having a viscosity of not more than 10 × 1 mm²/5 (10 centistockes) at 25°C).

Such products are commercially available from several manufacturers and include the tetrameric and pentameric dimethyl cyclosiloxane (also known as cyclomethicones) having the structures: 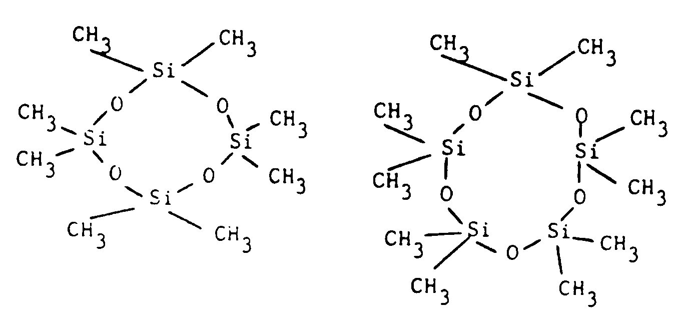
as well as linear compounds such as hexamethyl disiloxane (H₃C)₃Si―O―Si (CH₃)₃. The carrier comprises about 30 to about 70% of the total product, and preferably about 45 to about 65%. The carrier choice is cyclomethicone, which is a polydimethylcyclosiloxane.

The gelling agent is another essential ingredient of the invention, comprises about 7 to 30% of the total product, and preferably about 7 to 30% of the total product, and preferably about 7 to about 20%. Useful gelling agents include fatty alcohols having from 14 to 24 carbon atoms, hydrogenated castor oil, hydrogenated vegetable oil, fatty acids having from 14 to 36 carbon atoms, fatty acid ethylene glycol esters, glyceryl tribehenate, beeswax, paraffin wax or polyethylene. These gelling agents are available from many well-known chemical companies, including Emery, Vaschem Inc., Proctor & Gamble, Croda, Allied Chemical and others.

The gelling agent of preference is a combination of stearyl alcohol and hydrogenated castor oil, which is available as Castorwax MP-70 from Caschem. Castorwax MP-80 is a useful substitute for the MP-70. (Castorwax is a Registered Trade Mark).

The physiologically acceptable antiperspirant agent is the third essential ingredient of the invention, comprises about 12 to about 30% of the total product, and preferably about 18 to about 25%.

Antiperspirant compositions of necessity contain an effective antiperspirant agent. The number and composition of these agents is controlled in the United States by the Food and Drug Administration and the number of proposed agents is currently rather limited. They are in general aluminum or aluminumzirconium salts or complexes. See 47 Federal Register 36504, 20 August 1982. They include aluminum chloride, aluminum chlorohydrate, aluminum chlorohydrex, aluminum chlorohydrex PEG, aluminum chlorohydrex PG, aluminum dichlorohydrate, aluminum dichlorohydrex PEG, aluminum dichlorohydrex PG, aluminum sesquichlorohydrate, aluminum sesquichlorohydrex PEG, aluminum sesquichlorohydrex PG, aluminum sulfate, aluminum zirconium octachlorohydrate, aluminum zirconium octachlorohydrex GLY, aluminum zirconium pentachlorohydrate, aluminum zirconium pentachlorohydrex GLY, aluminum zirconium tetrachlorohydrate, aluminum zirconium trichlorohydrate, aluminum zirconium tetrachlorohydrate GLY, aluminum zirconium trichlorohydrate GLY. See CTFA Cosmetic Ingredient Dictionary, Third Editions, pp. 10-13, 279.

The term "physiologically acceptable" is used to mean a material which may be used in cosmetic-drug preparations for external use without violation of the appropriate regulations of the United States Food and Drug Administration.

The preferred physiologically acceptable antiperspirant agent is aluminum chlorohydrate. A suitable alternative to this is aluminum zirconium tetrachlorohydrex GLY (hereafter "Al―Zr TC GLY"). These agents are powders and have a preferred particle size of about 1 to about 100 microns.

Useful optional ingredients include surfactants which, when present, can comprise up to 5% of total product, and preferably about 2%. Useful surfactants will have hydrophilic-lipophilic balances (HLB) of about 10 to about 20. Nonionic surfactants with HLBs of 12-20 are preferred. The commercially available surfactant of choice is Arlacel-165, which is a mixture of glyceryl stearate and polyethylene glycol 100 stearate. Other useful surfactants are those of the Tween series (ethoxylated sorbitol monoesters having different degrees of ethoxylation to provide different HLBs), the Myrj series, the Brij series and the Atlas series. (Arlacel, Tween, Myrj, Brij and Atlas are Registered Trade Marks). All of these surfactants are commercially available from ICI United States and are well known.

Other useful optional ingredients are fillers, which, when present, can comprise up to about 20% of the total product and preferably about 10%. Useful fillers include talc, aluminum stearate, aluminum tristearate, zinc stearate, calcium carbonate or calcium stearate. Talc is preferred.

Other useful optional ingredients are emollients, which, when present, can comprise up to about 15% of the total products and preferably about 8%. An emollient is particularly useful when hydrogenated castor oil is used as the gelling agent or one of the gelling agents. The preferred emollient is fluid AP, which is commercially available and comprises the condensation product of about fourteen moles of propylene oxide with one mole of butanol (also known as polypropylene glycol 14 butyl ether).

Other useful optional ingredients are fragrances, which, when present, can comprise up to about 1% of the total product, and preferably about 0.5%.

Coloring agents are well known optional ingredients which may also be present in amounts of less than about 0.1%.

The following formulations exemplify the creamy products of the invention, all percentages being by weight of total product:

| Ingredient | Formulation | | |
|---|---|---|---|
| | 1 | 2 | 3 |
| Cyclomethicone | 52.5 | 54.0 | 59.80 |
| Stearyl alcohol | 24.0 | 7.2 | 6.25 |
| Castorwax MP-70 | 0.0 | 4.7 | 4.10 |
| Aluminum Chlorohydrate | 22.0 | 0.0 | 0.00 |
| Al-Zr TC GLY | 0.0 | 22.0 | 19.15 |
| Arlacel-165 | 1.0 | 1.0 | 1.00 |
| Fluid AP | 0.0 | 5.0 | 4.35 |
| Talc | 0.0 | 5.6 | 4.85 |
| Fragrance | 0.5 | 0.5 | 0.50 |

The method of the present invention comprises mixing and heating all ingredients except the fragrance, if any, to a temperature about the melting point of the gelling agent, cooling (while the mixing is continued) to approximately 55-65°., adding the fragrance, if any, while continuing to mix, then cooling further (while still mixing or agitating the product, or subjecting it to shear) to a temperature below the normal solidification point of the product, which is approximately in the range 44-52°C., for the indicated composition ranges. The product preferably is then charged into the push-up type applicators described more fully below, and is allowed to come to room temperature. The above method can be performed by hand using suitable heaters, mixers and cooling baths. The term "normal solidification point" us used herein means that temperature at which the product, when cooled without mixing or shearing, will ordinarily solidify to a block-type product.

In performing the method of the invention, it is preferred to use a commercial heat exchanger-mixer known as a Votator Scraped Surface Heat Exchanger to perform the step of cooling the product below the normal solidification point. This device is described more fully in bulletins available from one of the manufacturers, Chemetron Corporation, such as Bulletin V300. This device provides a very simple means for continuous thorough mixing of the product while it is being cooled to the desired temperature.

As will be readily apparent to those skilled in the art, the normal solidification point of the products of the invention will vary depending upon the actual carriers and gelling agents employed, and the amounts used of each.

It is believed that the method of the invention prevents the product from forming a solid matrix at its normal solidification point by continuous thorough mixing of the product as it cools to and below its normal solidification point. The product thus produced is a creamy heterogeneous, anhydrous antiperspirant suitable for packaging in jars or tubes. Another suitable package, and that preferred for the products of the invention, is a push-up type applicator, i.e. a tubular container having a piston advanced by a screw mechanism operated at the base thereof. The top of the tube may be closed by a perforated cap, with a cover being provided to seal the cap when not in use. Advance of the piston squeezes some of the product through the perforations onto the cap, whence it can be applied to the underarm.

It has also now been found that when the products of the invention are produced using the method of the invention, the products are creamy and do not readily separate. When the Standard Test Method for Cone Penetration of Petrolatum (ASTM D937-77) is performed on the products of the invention, the products exhibit a penentration value of about 10 to about 36 mm. Preferably, the products will exhibit a value of about 12 to about 20 mm.

When the same combinations of ingredients as those of the invention are prepared but are allowed to cool to below the normal solidification points without continuous agitation or mixing, the cone pentration values are much lower, namely, in the range of about 2 to about 8 mm.

## Claims

1. A creamy, anhydrous antiperspirant product comprising a volatile silicone carrier and an antiperspirant agent, characterised in that it is a clay-free, heterogeneous form comprising essentially about 30 to about 70% by weight of a volatile silicone product carrier, about 7 to about 30% by weight of a gelling agent and about 12 to about 30% by weight of a physioloically acceptable antiperspirant agent, and has a cone penetration value of about 10 to about 36 mm, and characterised in that it is obtainable by the steps of mixing and heating the carrier, the gelling agent, the antiperspirant agent and any optional ingredients except the fragrance (if any) to a temperature above the melting point of the gelling agent and then cooling the mixture below the normal solidification point of the product while still mixing or agitating the product or otherwise subjecting it to shear.

2. An antiperspirant product as claimed in claim 1, characterised in that the volatile silicone product carrier comprises about 45 to about 65% by weight of the total product.

3. An antiperspirant product as claimed in claim 1, characterised in that the gelling agent comprises about 7 to about 20% by weight of the total product.

4. An antiperspirant product as claimed in claim 1, characterised in that the physiologically acceptable antiperspirant agent comprises about 18 to about 25% by weight of the total product.

5. A creamy, anhydrous antiperspirant product comprising a volatile silicone carrier and an antiperspirant agent, characterised in that it is in a clay-free heterogeneous form consisting essentially of about 45 to about 65% by weight of a volatile silicone product carrier, about 7 to about 20% by weight of a gelling agent, about 18 to about 25% by weight of a physiologically acceptable antiperspirant agent and the remainder (if any) of optional ingredients selected from surfactants, emollients, fragrances, coloring agents, aluminum stearate, aluminum tristearate, zinc stearate, calcium stearate and calcium carbonate, and having a cone penetration value of about 10 to about 36 mm, and characterised in that is is obtainable by the steps of mixing and heating all the ingredients except the fragrance (if any) to a temperature above the melting point of the gelling agent and then cooling the mixture below the normal solidification point of the product while still mixing or agitating the product or otherwise subjecting it to shear.

6. An antiperspirant product as claimed in any of claims 1 to 5, characterised in that the carrier is a mixture of polydimethylcyclosiloxanes having 3 to 6 silicon atoms.

7. An antiperspirant product as claimed in any of claims 1 to 6, characterised in that the gelling agent is selected from fatty alcohols of 14-24 carbon atoms, hydrogenated castor oil, hydrogenated vegetable oil, fatty acids of 14 to 36 carbon atoms, fatty acid ethylene glycol esters, glyceryl tribehenate, beeswax, paraffin wax and polyethylene.

8. An antiperspirant product as claimed in any of claim 7, characterised in that the gelling agent is a combination of stearyl alcohol and hydrogenated castor oil.

9. An antiperspirant product as claimed in any of claims 1 to 8, characterised in that the physiologically acceptable antiperspirant agent is aluminum chlorohydrate or aluminum zirconium tetrachlorohydrex GLY.

10. An antiperspirant product as claimed in any of claims 1 to 9, characterised in that it additionally comprises up to 5% by weight of a nonionic surfactant having an HLB value of about 10 to about 20.

11. An antiperspirant product as claimed in claim 10, characterised in that it additionally comprises up to 2% by weight of a nonionic surfacant having an HLB value of about 10 to about 20.

12. An antiperspirant product as claimed in claim 10 to 11, characterised in that the surfactant is a mixture of glyceryl stearate and polyethylene glycol 100 stearate.

13. An antiperspirant product as claimed in any of claims 1 to 12, characterised in that it additionally comprises up to about 15% by weight of an emollient.

14. An antiperspirant product as claimed in claim 13, characterised in that it additionally comprises up to about 8% by weight of an emollient.

15. An antiperspirant product as claimed in claim 13 or 14, characterised in that the emollient is polypropylene glycol 14 butyl ether.

16. An antiperspirant product as claimed in any of claims 1 to 4 or in claim 6, 7, 9, 10 or 13 as appendant to any of claims 1 to 4, characterised in that it additionally comprises up to 5.6% by weight of talc.

17. A method of making a creamy, clay-free, heterogeneous, anhydrous antiperspirant product as claimed in any of claims 1 to 16, characterised by the steps of mixing and heating the carrier, the gelling agent and the antiperspirant agent to a temperature above the melting point of the gelling agent, and cooling while the mixing is continued to a temperature below the normal solidification point of the product.

18. A method as claimed in claim 17 of making a fragrant, creamy, clay-free, heterogeneous, anhydrous antiperspirant product, characterised by the steps of mixing and heating the carrier, the gelling agent, the antiperspirant agent and any optional ingredients except the fragrance to a temperature above the melting point of the gelling agent, cooling (while the mixing is continued) to approximately 55-65°C, adding the fragrance while continuing to mix, then cooling further while still mixing the product to a temperature below the normal solidification point of the product.

## Patentansprüche

1. Cremiges, wasserfreies, schweißverhütendes Produkt bestehend aus einem leicht flüchtigen Silikonträger und einem Schweißverhütungsmittel, dadurch gekennzeichnet, daß es in tonfreier, heterogener Form vorliegt, im wesentlichen aus etwa 30 bis etwa 70 Gew.-% eines leicht flüchtigen Silikonträgers, etwa 7 bis etwa 30 Gew.-% eines Geliermittels und etwa 12 bis etwa 30 Gew.-% eines physiologisch annehmbaren Schweißverhütungsmittels besteht und einen Konuspenetrationswert von etwa 10 bis etwa 36 mm aufweist und dadurch gekennzeichnet, daß es dadurch erhältlich ist, daß man den Träger, das Geliermittel, das Schweißverhütungsmittel und gegebenenfalls weitere Bestandteile, außer dem Duftstoff, vermischt und auf eine Temperatur oberhalb des Schmelzpunktes des Geliermittels erhitzt und die Mischung sodann bei gleichzeitiger Fortsetzung des Misch- oder Rührvorganges oder einer anderen Scherbehandlung des Produkts auf eine Temperatur unterhalb des normalen Verfestigungspunktes des Produkts abkühlen läßt.

2. Schweißverhütendes Produkt nach Anspruch 1, dadurch gekennzeichnet, daß der leicht flüchtige Silikonträger aus etwa 45 bis etwa 65 Gew.-% des Gesamtprodukts besteht.

3. Schweißverhütendes Produkt nach Anspruch 1, dadurch gekennzeichnet, daß das Geliermittel aus etwa 7 bis etwa 20 Gew.-% des Gesamtprodukts besteht.

4. Schweißverhütendes Produkt nach Anspruch 1, dadurch gekennzeichnet, daß das physiologisch annehmbare Schweißverhütungsmittel aus etwa 18 bis etwa 25 Gew.-% des Gesamtprodukts besteht.

5. Cremiges, wasserfreies, schweißverhütendes Produkt bestehend aus einem leicht flüchtigen Silikonträger und einem Schweißverhütungsmittel, dadurch gekennzeichnet, daß es in tonfreier, heterogener Form vorliegt und im wesentlichen aus etwa 45 bis etwa 65 Gew.-% eines leicht flüchtigen Silikonträgers, etwa 7 bis etwa 20 Gew.-% eines Geliermittels und etwa 18 bis etwa 25 Gew.-% eines physiologisch annehmbaren Schweißverhütungsmittels und der Rest (falls vorhanden) aus nicht erforderlichen Bestandteilen, ausgewählt aus Tensiden, erweichenden Mitteln, Duftstoffen, Farbmitteln, Aluminiumstearat, Aluminiumtristearat, Zinkstearat, Calciumstearat und Calciumkarbonat besteht, wobei jenes schweißverhütende Produkt einen Konuspenetrationswert von etwa 10 bis etwa 36 mm aufweist und dadurch gekennzeichnet, daß es dadurch erhältlich ist, daß man alle Bestandteile, außer dem Duftstoff (falls vorhanden), vermischt und auf eine Temperatur oberhalb des Schmelzpunktes des Geliermittels erhitzt und die Mischung sodann bei gleichzeitiger Fortsetzung des Misch- oder Rührvorganges oder einer anderen Scherbehandlung des Produkts auf eine Temperatur unterhalb des normalen Verfestigungspunktes des Produkts abkühlen läßt.

6. Schweißverhütendes Produkt nach einem der Ansprüche 1 bis 5, dadurch gekennzeichnet, daß es sich bei dem Träger um eine Mischung von Polydimethylcyclosiloxanen mit 3 bis 6 Siliziumatomen handelt.

7. Schweißverhütendes Produkt nach einem der Ansprüche 1 bis 6, dadurch gekennzeichnet, daß das Geliermittel aus Fettalkoholen mit 14 - 24 Kohlenstoffatomen, gehärtetem Rizinusöl, gehärtetem Pflanzenöl, Fettsäuren mit 14 bis 36 Kohlenstoffatomen, Fettsäureethylenglykolestern, Glycerintribehenat, Bienenwachs, Paraffinwachs und Polyethylen ausgewählt ist.

8. Schweißverhütendes Produkt nach Anspruch 7, dadurch gekennzeichnet, daß es sich bei dem Geliermittel um eine Kombination von Stearylalkohol und gehärtetem Rizinusöl handelt.

9. Schweißverhütendes Produkt nach einem der Ansprüche 1 bis 8, dadurch gekennzeichnet, daß es sich bei dem physiologisch annehmbaren Schweißverhütungsmittel um Aluminiumchlorhydrat oder Aluminiumzirkoniumtetrachlorhydrex GLY handelt.

10. Schweißverhütendes Produkt nach einem der Ansprüche 1 bis 9, dadurch gekennzeichnet, daß es zusätzlich bis zu 5 Gew.-% eines nichtionischen Tensids mit einem HLB-Wert von etwa 10 bis etwa 20 enthält.

11. Schweißverhütendes Produkt nach Anspruch 10, dadurch gekennzeichnet, daß es zusätzlich bis zu 2 Gew.-% eines nichtionischen Tensids mit einem HLB-Wert von etwa 10 bis etwa 20 enthält.

12. Schweißverhütendes Produkt nach Anspruch 10 oder 11, dadurch gekennzeichnet, daß es sich bei dem Tensid um eine Mischung aus Glycerinstearat und Polyethylenglykol-100-stearat handelt.

13. Schweißverhütendes Produkt nach einem der Ansprüche 1 bis 12, dadurch gekennzeichnet, daß es zusätzlich bis etwa 15 Gew.-% eines erweichenden Mittels enthält.

14. Schweißverhütendes Produkt nach Anspruch 13, dadurch gekennzeichnet, daß es zusätzlich bis etwa 8 Gew.-% eines erweichenden Mittels enthält.

15. Schweißverhütendes Produkt nach Anspruch 13 oder 14, dadurch gekennzeichnet, daß es sich bei dem erweichenden Mittel um Polypropylenglykol-14-butylether handelt.

16. Schweißverhütendes Produkt nach einem der Ansprüche 1 bis 4 oder nach Anspruch 6, 7, 9, 10 oder 13, soweit auf einen der Ansprüche 1 bis 4 rückbezogen, dadurch gekennzeichnet, daß es zusätzlich bis zu 5,6 Gew.-% Talk enthält.

17. Verfahren zur Herstellung eines cremigen, tonfreien, heterogenen, wasserfreien, schweißverhütenden Produkts nach einem der Ansprüche 1 bis 16, dadurch gekennzeichnet, daß man den Träger, das Geliermittel und das Schweißverhütungsmittel vermischt und auf eine Temperatur oberhalb des Schmelzpunktes des Geliermittels erhitzt und bei gleichzeitiger Fortsetzung des Mischvorganges auf eine Temperatur unterhalb des normalen Verfestigungspunktes des Produkts abkühlen läßt.

18. Verfahren nach Anspruch 17 zur Herstellung eines duftenden, cremigen, tonfreien, heterogenen, wasserfreien, schweißverhütenden Produkts, dadurch gekennzeichnet, daß man den Träger, das Geliermittel, das Schweißverhütungsmittel und gegebenenfalls weitere Bestandteile außer dem Duftstoff vermischt und auf eine Temperatur oberhalb des Schmelzpunktes des Geliermittels erhitzt, auf etwa 55 - 65°C (bei gleichzeitiger Fortsetzung des Mischvorganges) abkühlen läßt, den Duftstoff bei gleichzeitiger Fortsetzung des Mischvorganges zugibt, sodann weiter auf eine Temperatur unterhalb des normalen Verfestigungspunktes des Produkts abkühlen läßt, wobei man das Produkt immer noch mischt.

## Revendications

1. Produit antitranspirant anhydre crémeux, comprenant un véhicule silicone volatil et un agent antitranspirant, caractérisé en ce qu'il s'agit d'une forme hétérogène sans argile comprenant essentiellement d'environ 30 à environ 70% en poids d'un véhicule à base de produit silicone volatil, d'environ 7 à environ 30% en poids d'un agent gélifiant volatil et d'environ 12 à environ 30% en poids d'un agent antitranspirant physiologiquement acceptable, et qu'il a un indice de pénétration au cône d'environ 10 à environ 36 mm, et caractérisé en ce que l'on peut l'obtenir par les étapes de mélange et de chauffage du véhicule, de l'agent gélifiant, de l'agent antitranspirant et de tout ingrédient facultatif, à l'exception du parfum (éventuel), à une température supérieure au point de fusion de l'agent gélifiant, puis de refroidissement du mélange au-dessous du point de solidification normal du produit, tout en mélangeant ou en agitant encore le produit, ou en le soumettant à un cisaillement autre.

2. Produit antitranspirant selon la revendication 1, caractérisé en ce que le véhicule à base de produit silicone volatil constitue d'environ 45 à environ 65% en poids du produit total.

3. Produit antitranspirant selon la revendication 1, caractérisé en ce que l'agent gélifiant constitue d'environ 7 à environ 20% en poids du produit total.

4. Produit antitranspirant selon la revendication 1, caractérisé en ce que l'agent antitranspirant physiologiquement acceptable constitue d'environ 18 à environ 25% en poids du produit total.

5. Produit antitranspirant anhydre crémeux, comprenant un véhicule silicone volatil et un agent antitranspirant, caractérisé en ce qu'il se présente sous une forme hétérogène sans argile essentiellement constituée d'environ 45 à environ 65% en poids d'un véhicule à base de produit silicone volatil, d'environ 7 à environ 20% en poids d'un agent gélifiant volatil, d'environ 18 à environ 25% en poids d'un agent antitranspirant physiologiquement acceptable et le reste (éventuel) d'ingrédients facultatifs choisis parmi les tensioactifs, les émollients, les parfums, les agents colorants, le stéarate d'aluminium, le tristéarate d'aluminium, le stéarate de zinc, le stéarate de calcium et le carbonate de calcium, et ayant un indice de pénétration au cône d'environ 10 à environ 36 mm, et caractérisé en ce que l'on peut obtenir par les étapes de mélange et de chauffage de tous les ingrédients, a l'exception du parfum (éventuel), à une température supérieure au point de fusion de l'agent gélifiant, puis de refroidissement du mélange au-dessous du point de solidification normal du produit, tout en mélangeant ou en agitant encore le produit, ou en le soumettant à un cisaillement autre.

6. Produit antitranspirant selon l'une quelconque des revendications 1 à 5, caractérisé en ce que le véhicule est un mélange de polydiméthylcyclosiloxanes comportant 3 à 6 atomes de silicium.

7. Produit antitranspirant selon l'une quelconque des revendications 1 à 6, caractérisé en ce que l'agent gélifiant est choisi parmi les alcools gras de 14-24 atomes de carbone, l'huile de ricin hydrogénée, une huile végétale hydrogénée, les acides gras de 14 à 36 atomes de carbone, les esters d'éthylèneglycol et d'acides gras, le tribéhénate de glycéryle, la cire d'abeille, la cire de paraffine et le polyéthylène.

8. Produit antitranspirant selon la revendication 7, caractérisé en ce que l'agent gélifiant est une combinaison d'alcool stéarylique et d'huile de ricin hydrogénée.

9. Produit antitranspirant selon l'une quelconque des revendications 1 à 8, caractérisé en ce que l'agent antitranspirant physiologiquement acceptable est le chlorohydrate d'aluminium ou le tétrachlorohydrex d'aluminium et de zirconium GLY.

10. Produit antitranspirant selon l'une quelconque des revendications 1 à 9, caractérisé en ce qu'il comprend en outre jusqu'à 5% en poids d'un tensioactif non ionique ayant un rapport hydro-lipophile d'environ 10 à environ 20.

11. Produit antitranspirant selon la revendication 10, caractérisé en ce qu'il comprend en outre jusqu'à 2% en poids d'un tensioactif non ionique ayant un rapport hydrolipophile d'environ 10 à environ 20.

12. Produit antitranspirant selon la revendication 10 ou 11, caractérisé en ce que le tensioactif est un mélange de stéarate de glycéryle et de stéarate de polyéthylèneglycol 100.

13. Produit antitranspirant selon l'une quelconque des revendications 1 à 12, caractérisé en ce qu'il comprend en outre jusqu'à environ 15% en poids d'un émollient.

14. Produit antitranspirant selon la revendication 13, caractérisé en ce qu'il comprend en outre jusqu'à environ 8% en poids d'un émollient.

15. Produit antitranspirant selon la revendication 13 ou 14, caractérisé en ce que l'émollient est l'éther butylique de polypropylèneglycol 14.

16. Produit antitranspirant selon l'une quelconque des revendications 1 à 4 ou la revendication 6, 7, 9, 10 ou 13 dépendant de l'une quelconque des revendications 1 à 4, caractérisé en ce qu'il comprend en outre jusqu'à 5,6% en poids de talc.

17. Procédé de fabrication d'un produit antitranspirant anhydre, hétérogène, sans argile, crémeux, selon l'une quelconque des revendications 1 à 16, caractérisé par les étapes de mélange et de chauffage du véhicule, de l'agent gélifiant et de l'agent antitranspirant jusqu'à une température supérieure au point de fusion de l'agent gélifiant et de refroidissement, tout en poursuivant le mélange, jusqu'à une température inférieure au point de solidification normal du produit.

18. Procédé selon la revendication 17 de fabrication d'un produit antitranspirant anhydre, hétérogène, sans argile, crémeux, parfumé, caractérisé par les étapes de mélange et de chauffage du véhicule, de l'agent gélifiant et de l'agent antitranspirant et de tous les ingrédients facultatifs éventuels, à l'exception du parfum, jusqu'à une température supérieure au point de fusion de l'agent gélifiant, de refroidissement (tout en poursuivant le mélange) jusqu'à environ 55-65°C, d'addition du parfum tout en poursuivant le mélange, puis de refroidissement supplémentaire, tout en poursuivant le mélange, jusqu'à une température inférieure au point de solidification normal du produit.
